# EUROPEAN PATENT SPECIFICATION

(11) **EP 3 168 860 B1**
(45) Date of publication and mention of the grant of the patent: **19.12.2018**
(21) Application number: 16002252.1
(22) Date of filing: 19.10.2016
(51) Int. Cl.: H01J 61/12

(54) **DEVICE AND METHOD FOR PRODUCING UV RADIATION**
VORRICHTUNG UND VERFAHREN ZUR ERZEUGUNG VON UV-STRAHLUNG
DISPOSITIF ET PROCÉDÉ DE PRODUCTION DE RAYONNEMENT UV

(30) Priority: 16.11.2015 CZ 20150815
(43) Date of publication of application: 17.05.2017
(73) Proprietor: Tomas Bata University In Zlín, 76001 Zlín (CZ); Institut "Jozef Stefan", 1000 Ljubljana (SI)
(72) Inventor: Lehocký, Marián, 76001 Zlín (CZ); Stloukal, Petr, 76005 Zlín (CZ); Sedlarík, Vladimír, 76302 Zlín-Malenovice (CZ); Humpolícek, Petr, 76861 Bystrice pod Hostýnem (CZ); Vesel, Alenka, 820 Trebnje (SI); Mozetic, Miran, 1251 Moravce (SI); Zaplotnik, Rok, 1000 Ljubljana (SI); Primc, Gregor, 1381 Rakek (SI)
(74) Representative: Kreizlova, Dana

(56) References cited:
- CN-U- 204 216 007
- JP-A- H11 329 368
- MILLER H C ET AL: "An optically pumped ultraviolet laser on SO (B<3>[Sigma]<->-X<3>[Sigma]<->)", CHEMICAL PHYSICS LETTERS, vol. 181, no. 2-3, 21 June 1991 (1991-06-21), pages 250-254, XP55352991, ISSN: 0009-2614

## Description

### FIELD OF THE INVENTION

The current invention relates to the device for producing of ultraviolet (UV) radiation as well as the method for producing such UV radiation. It is in particular UV A and UV B radiation with negligible amount of radiation in the visible and infra-red range. The device and the method for producing the UV radiation according to the present invention is applicable for many treatments of materials such as destruction of bacteria, viruses, spores, fungi and eventual sterilization of heat-sensitive materials as well as UV curing of thin layers of heat-sensitive materials.

### BACKGROUND OF THE INVENTION

The effects of UV radiation are currently being used for various applications such as activation of polymer materials prior to painting, printing, gluing or deposition of various coatings, sterilization of different objects including devices and components used in medical praxis, and curing of polymers in order to improve the scratch resistance. Depending on particular application the suitable range of UV radiation could be either UV A (from about 320 to 400 nm) or UV B (from about 280 to 320 nm) or UV C (from about 200 to 280 nm), or combination of two or more types of UV radiation. Radiation at lower wavelength is called VUV (vacuum ultra violet).

Another classification divides UV radiation to near UV (300 - 400 nm), middle UV (200 - 300 nm), far UV (122 - 200 nm) and extreme UV (from 10 to 122 nm).

Many practical utilisations employ near UV radiation (UV A and a part of UV B) since the photon energy does not allow for dissociation of oxygen molecules in air and consequently production of harmful ozone. Best germicidal effects, on the other hand, are obtained using middle range UV (UV C and B) while curing of polymer thin films is accomplished using both near and middle range UV radiation. The radiation in the far UV and VUV range is limited in practice since the radiation is absorbed by air molecules. Furthermore, inexpensive materials for UV bulbs such as quartz glass are transparent only for wavelengths above about 200 nm. For all mentioned applications the ideal solution would be a lamp of high energy efficiency and broad continuum of UV radiation covering both UV A and UV B and at least a part of the UV C range. Also, the ideal source will have a prolonged life time, currently limited to a few 1000 hours for standard mercury lamps.

Classical UV lamps employ mercury vapour. Mercury is evaporated using an appropriate method and excited by passing electrical current trough the mercury vapour. The excited mercury atoms relax to the ground state by electrical dipole radiation so they produce a rather intensive radiation in both visible and ultra violet (UV) range of spectrum. Mercury lamps are used widely nowadays despite the fact that they represent an environmental hazard if used bulbs are not disposed properly. Another disadvantage of the mercury lamps is energy efficiency since only a small fraction of energy is transferred to UV radiation. The majority of energy is spent for heating of the bulb and a significant fraction also for radiation in the visible range.

Therefore there is a need for optimization of the UV sources to be ecologically benign and of high energy efficiency. Novel devices as sources of UV radiation have been subjects of numerous patent applications as well as granted patents.

The first group of innovative UV sources is based on solid emitting tools, such as light emitting diode (LED) or fluorescent coating.

For example, CN 102563453 discloses a complex device suitable for delivering UV radiation in a broad range of wavelengths. Light source has one or more solid state UV emitters for emitting light at single wavelengths in the range of 240 to 400 nm. The light emitted by each of the emitters has a bandwidth in the order of 10-20 nm and is directed toward a solid material. The UV emitters are enclosed in a housing which can be attached to any material including sensors. The disadvantage of such UV source lies primarily in the fact that its intensity decreases during the time of using.

Chines UV source disclosed as CN202501224 utility model embodiment of an ultraviolet light emitting diode (UV LED) light source which is used to high-efficiently solve a heat-dissipation problem of a UV LED circuit. The UV LED light source comprises a fixing substrate, the UV LED circuit and a water cooling apparatus. The fixing substrate is used to fix the UV LED circuit and the water cooling apparatus. The water cooling apparatus comprises a water circulation heat conduction channel. The UV LED circuit comprises a UV LED packaging module. A heat conduction module of the UV LED packaging module is contacted with the water circulation heat conduction channel. The said kind of UV source shows the same disadvantage as the previous one - its intensity falls down during the time.

Another protected solution CN202902229 discloses an intelligent UV power source radiator which comprises a housing and a power board installed inside the housing. Radiating fins are arranged on the lower surface of the power board, a fan is arranged on the surface of the radiating fin, and a ventilation slot is reserved in the housing. Through the radiating fin arranged on the lower surface of the power board, the fan arranged on the surface of the radiating fin, and the ventilation slot reserved in the housing, heat generated from operating the UV power source is timely dissipated by the radiating fins and sent to the air through the fan which is stuck on the surface of the radiating fin and the ventilation slot in the housing, so that the cooling function of the UV power source is improved, and the service life of the UV power source is prolonged. However, the described UV power source is rather complicated, moreover, the time of using of the individual discharge is limited.

CN203176741 teaches a mercury free source of UV radiation of prolonged life-time at about 10000 hours. The UV excitation system comprises an electronic gun used for generating an electron beam and a fluorescent screen, wherein the fluorescent screen is provided with a light-permeable fluorescent screen surface, UV fluorescence powder used for emitting UV radiation is coated on the fluorescent screen surface to form a fluorescence powder layer, and the electronic gun faces the fluorescent screen surface. The UV excitation system adopting the electron beam to excite the fluorescent UV source can provide several watts of irradiation power, and the spectral line pulse width is guaranteed by the characteristics of the fluorescence powder. The need of the said fluorescence powder is the main disadvantage of the said UV source.

CN 203288562 teaches a flat UV radiation light source triggered by field emission array comprising an anode plate. The anode plate is provided with a UV fluorescent powder layer, and the light source also comprises a field emission cathode array, which faces the anode plate. An electron beam can be generated by adopting the field emission cathode array, and the UV fluorescent powder of the UV fluorescent powder layer can be used to emit the ultraviolet light. Compared to the traditional UV light sources, the flat UV radiation light source has advantages of mercury-free performance, energy saving, long service lifetime, and good illumination quality. Compared to the UV light source triggered by the traditional thermal emission electronic gun, the flat UV radiation light source is advantageous in that the appearance is thin, the light source can be used for different purposes, and the field emission structure design is suitable for the large-scale production. The drawback of it is the same as for the previous one - the need of the fluorescence powder.

WO 2015038433 discloses a complex light-emitting source for curing applications. The light-emitting source comprises a first housing having a top wall and one or more side walls. The top wall and the one or more side walls define a first enclosure having a first open end. The light-emitting source further comprises a plurality of light-emitting devices arranged within the first enclosure of the first housing. One side of each of the plurality of light-emitting devices faces outward from the first open end of the first enclosure. The plurality of light-emitting devices is configured to emit light from the first open end to produce a substantially uniform area of illumination on a facing portion of a surface of a target. The described device is rather complicated and accordingly its using with time limiting can be reasonably expected.

Patent application JP 2009054460 teaches a method for selecting an appropriate range of UV radiation. The light source for UV irradiation comprises a UV irradiation lamp; and a silicate film which absorbs a wavelength composition of a part of light generated by the UV irradiation lamp and contains one or more types of metal oxides. The wavelength of absorbed light can be varied depending on types and a compounding ratio of the selected metal. The certain drawback can be seen in the need of using a silicate film and in the limited extent of wavelength of absorbed light depending on used types of metal oxides.

The devices using solid UV sources are advantageous from the point of view providing of continuous radiation. However, their common drawback is that they can provide UV radiation mainly with the limited extent of wavelengths.

Exemplary device which provides light from light sources that operate in accordance with different physical principles, can be here mentioned: WO2014060592 discloses a complex device comprising a chamber having several gas-filled plasma chambers, wherein the chamber has at least one area transparent to UV light and/or VUV light. A first group of plasma chambers is filled with an ionisable gas containing mercury and a second group of plasma chambers is filled with a gas that forms excimers when suitably excited. The device is therefore suitable for employing radiation from different atoms including mercury. The general disadvantage of the described device is - despite its above mentioned assets - environmental hazard connected with mercury using.

Parallel to above described group of UV sources based on solid emitters nowadays there is another group of UV sources based on various kinds of atoms.

An exemplary solution according to JP2012222260 discloses a source of UV radiation with strong radiation in the extreme UV range. The device comprises a tube with positive and negative electrodes, and a suitable DC power source allows for creation of gaseous plasma in the tube between the electrodes. The tube is filled with predetermined atoms which are excited by plasma electrons and emit light by dipole radiation. The electrical current through the device is large so the electrodes are heated during operation so the cooling by flowing water is essential. The same fact can be perceived as significant disadvantage of this UV source.

WO0109924 discloses an ultraviolet light source comprising an ultraviolet lamp, a microwave energy source for exciting said ultraviolet lamp and an enclosure for enclosing the ultraviolet lamp, the enclosure comprising an optically transparent waveguide. The ultraviolet bulb has no electrode. The bulb is partially evacuated tube comprising an element or mixtures of elements in vapour form. Mercury is the preferred element but alternatives include mixtures of inert gas with mercury compounds, sodium or sulphur. Halides and amalgams are also suitable. The certain drawback of such UV lamp lies again in hazardous mercury using.

US2012248301 discloses a UV-LED photoemission ionization source. It provides ionization of analytes including volatile molecular species and organic residues. The UV-LED source produces low-energy UV light (200 nm to 400 nm) that yields photoemission electrons from various conducting surfaces. These photoemission electrons provide direct and indirect ionization of analytes including trace organic residues without need of high electric fields. The source described is directed on the destruction of residual gaseous waste organic molecules; it does not allow the curing, therefore it is not suitable for UV curing of thin layers and further similar use.

DE10044655 discloses an ion source which has an ionisation chamber and a UV/VUV-excimer light source for generating ions by directing light into a sample gas. The light source is provided by a deuterium lamp, a micro hollow cathode lamp, a micro point lamp, a DC discharge lamp, a barrier discharge lamp, or an electron beam UV/VUV lamp, with an electron gun, a membrane between the electron gun and a gas space containing a rare earth gas or gas mixture and optical elements for imaging the light emission volume into the ionisation space. This device of quite complicated structure has its drawback also taking into account the need of earth gas.

KR100832398 discloses a non-electrode zirconium ultraviolet lamp and a liquid sterilizing device using the same. Germs in water are deactivated by lighting ultraviolet rays having main wavelength of 262.1nm. A liquid sterilizing device includes a plural ultraviolet ray emitting portion and a couple of resonance devices. The ultraviolet ray emitting portion includes an ultraviolet ray lamp and a protection pipe. The couple of resonance devices include a super-high frequency generator and waveguides. Both ends of the lamp tube portion are bonded tightly to the open holes of the couple of resonance devices. An air provider is installed on the upper part of a waveguide of the resonance devices. A diffuser that communicates with the waveguide and has a diffusing nozzle mounted thereon is installed on the lower part of the waveguides. The weak point of the device regarding its using is the fact that it is not able of curing of square or spatial structures.

US5191460 discloses a UV source which includes an electrodeless capsule of UV transmitting material filled with iodine vapour. A means of energizing the iodine vapour into a plasma state is provided by a magnetron, power triode or solid state supply. A circuit to modulate the energizing means is used to impress upon the plasma discharge the encoded data. Filtering means surround the capsule to filter out the plasma background noise. The advantages of the method and apparatus are increased pulse rate, faithful conversion of electrically pulsed data into optical pulses with decreased error rate, decreased probability of interception and non-line of sight communication. The described UV source is interesting from the point of view quite different kind of source comparing to the sources mentioned earlier. However, its drawback is the using of iodine.

DE19613357 discloses a pulsed light source with a lamp head and a gas container resistant to the reaction gas and the light radiation. A gas discharge can be generated in the container. The device also has at least a first electrode, e.g. cathode electrode or mains electrode, shaped to provide a wide discharge area. The electrode is made of chemically and electrically resistant material and is electrically connected to the gas container. The device also has at least a second electrode e.g. an anode electrode shaped to provide a wide discharge area and which is electrically connected to a positive potential of a high voltage source. An electrode spacer e.g. a lamp chamber is provided such that a discharge area is formed between the electrodes when the lamp is operable. An automatic ignition device generates a directed and homogenous gas discharge between the electrodes. A high voltage circuit generates the necessary high voltage pulse with a rapid rate of increase. At least one light outlet window which allows light from the lamp through and which is chemically resistant, is provided in the wall of the lamp chamber or the container. A control system monitors and regulates all required operating values of the lamp and gas collection. A gas or gas mixture filling is provided for the respective light emission. A variety of gases are suitable, preferably noble gases at pressure from 0.1 to 20 bar. The complicated structure of the solution is evident.

The article "An optically pumped ultraviolet laser on SO (B3∑- - X3 ∑-)" by H C Miller et al. in Chem. Phys. 181 (2-3), pages 250-254 (1991; ISSN: 0009-2614), discloses a UV laser with a radiation source based on SO molecules according to the preamble of claim 1. There, however, the SO is created within the laser tube from SO by irradiation with 193 nm (ArF excimer) and additional pumping with 256 nm radiation.

The UV sources according patents cited above teaching methods or devices based on atomic species can eliminate or reduce some of disadvantages of previous ones, they can provide broader extent of wavelength. Their weakness is their discrete radiation and their relatively low efficiency. On the other hand the greater range of wavelengths reached usually includes the complex UV radiation with certain amount of radiation in the visible or near IR range, therefore it can be difficult or impossible to avoid the overheating of treated surfaces, which is undesirable particularly regarding heat-sensitive materials.

### BRIEF DESCRIPTION OF THE INVENTION

Above mentioned drawbacks and deficiencies regarding current devices for producing of UV radiation are mainly reduced or eliminated using a device for producing of ultraviolet (UV) radiation according to the present invention. The ground of the invention is based on the fact that the device is equipped by means for a continuous supply of sulphur oxide molecules with a content of free electrons into said luminous tube, such that the said luminous tube contains a stable amount of sulphur oxide (SO) molecules, and a stable amount of free electrons. The device according to the invention can have the luminous tube of a linear shape and closed at its ends by vacuum flanges which are equipped by either input or outlet opening for sulphur oxide with the content of free electrons. The linear luminous tube can contain a coaxially placed inserted tube which passes through at least one vacuum flange wherein the walls of the said inserted tube are transparent for UV A and UV B radiation, and the device is so adapted that object can be placed inside the inserted tube, the said object is intended for the treatment by UV A and UV B radiation. This inserted tube can pass also through both of opposite vacuum flanges wherein transporting rollers are placed near to vacuum flanges, the said transporting rollers are adapted for movement of the object with longitudinal dimension exceeding the relevant dimension of the device, inside the inserted tube.

The device according to the invention can have more luminous tubes, advantageously a set of luminous tubes which are adapted to be spaced around the object intended for the treatment by UV A and UV B radiation. The luminous tubes can be not only linear but for example of a toroidal shape.

The device according to the invention is equipped advantageously with a reflective casing which is created from UV radiation reflecting material.

The ground of the method according to the invention is based on following steps: the luminous tube is filled by sulphur oxide (SO) molecules with stable amount of free electrons, wherein the SO molecules are supplied and drained inside/outside the luminous tube in such a way that the pressure inside the luminous tube as well as the amount of free electrons remains constant.

During the method according to the invention the luminous tube can contain SO molecules with temperature between 200 and 600 K, preferably between 300 and 350 K, and with constant pressure between 1 and 10000 Pa, preferably between 30 and 100 Pa. Free electrons present in the luminous tube can have the average kinetic energy between 0.1 and 10 eV, preferably between 2 and 8 eV. These free electrons can have the density between 10¹⁴ and 10¹⁸ m⁻³, preferably between 10¹⁵ and 10¹⁷ m⁻³.

The principal advantage of the device according to the invention is almost complete elimination of adjacent thermal effect which is typical for current UV sources causing the simultaneous heating of the material being treated by UV radiation.

The present invention is distinguished from common approaches by utilizing double-atom molecules suitable for excitation by low-energy electrons (typically between 2 and 6 eV) and capable of emitting UV radiation at transition from the excited to the ground state. The list of suitable double-atom molecules is limited since many molecules such as N₂, O₂, H₂, F₂, Cl₂ and alike do not satisfy the upper criteria due to the rules of quantum mechanics. The exception is hydrogen molecule that is capable of emitting continuous radiation if excited to the highly energetic state but the dissociation to parent atoms prohibits high energy efficiency of such UV sources. Namely, the majority of electron energy is spent for dissociation and ionization of molecules as well as excitation of atoms which emit light either in the visible range (Balmer series) or extreme UV range (Lyman series).

For the device and the method according to the invention the sulphur monoxide (SO) molecules are used as the source of intensive UV radiation. A suitable chamber made from a material transparent for the UV A, UV B and UV C radiation (quartz glass in the preferred embodiment) is filled with SO molecules which are excited by low energy electrons, typically with a Maxwellian energy distribution function peaking at the energy of about 4 eV. Such electrons are capable of exciting SO molecules to radiative states but the probability for electron impact dissociation to parent atoms is small. The majority of electron energy is thus spent for excitation of SO molecules to the radiative states rather than dissociation. The energy efficiency of the UV source according to the invention is superior as compared to known gaseous sources of UV radiation.

### BRIEF DESCRIPTION OF FIGURES

The exemplary embodiments of the invention are depicted using the set of drawings comprising 7 figures that show:
- Figure 1: a device for producing UV radiation according to the invention appropriate for treatment of small objects in batch process;
- Figure 2: a device for producing UV radiation according to the invention suitable for treatment of infinite objects in continuous process;
- Figure 3: a device for producing UV radiation according to the invention determined for treatment of large objects in either batch or continuous process;
- Figure 4: an alternative embodiment of the device for producing UV radiation according to the invention, intended for treatment of large objects in either batch or continuous process;
- Figure 5: comparative - spectrum of a commercial UV source for curing polymers;
- Figure 6: comparative - temperature of a thin layer of a UV cured coating based on acrylate deposited on the surface of styrene acrylonitrile sheet (SAN) by a print coat instrument upon curing with a commercial UV source for curing polymers;
- Figure 7: spectrum of a device for producing UV radiation according to the invention.

### DETAILED DESCRIPTION OF THE INVENTION - EXAMPLES

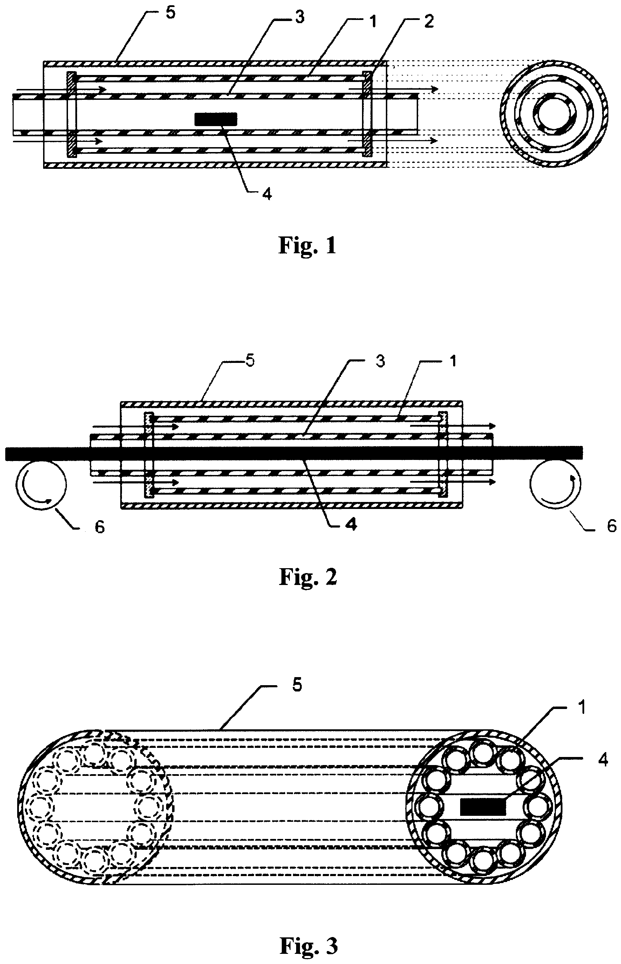

The crucial part of the device according to the invention is the luminous tube 1 (see Fig. 1 - 4) either in simple or multiple embodiment, which contains stable amount of sulphur oxide (SO) molecules, and stable amount of free electrons. The vigorous medium for the producing of UV radiation in the luminous tube 1 is permanently assured through the vacuum flange 2 by filling the evacuated luminous tube 1 with SO molecules and continuous supplying the same. The SO molecules are continuously excited by electrons which are also presented in their volume. The present SO molecules are simultaneously drained away through the opposite vacuum flange 2'. The relevant temperature and pressure values of SO molecules for individual embodiments as well as the values of free electron density are mentioned in following examples.

### Example 1

An embodiment according to the example 1 is appropriate for producing UV radiation for treatment of small objects in batch process as it is schematically shown in the Fig. 1. A luminous tube 1 has linear shape and it is made from the material transparent for UV radiation, namely from quartz glass, and it is at both of its ends tightly connected to a vacuum flanges 2, 2', which are equipped by a closable inlet/output opening intended for the evacuation and filling/draining of SO molecules with free electron content. There is an inserted tube 3 coaxially placed into the luminous tube 1 wherein the inserted tube 3 diameter is 100 mm lower than that one of luminous tube 1. The inserted tube 3 is made from the material transparent for UV radiation, namely from quartz glass, and it is at both of its ends tightly connected to a vacuum flanges 2, 2', thus the pressure inside the inserted tube 3 corresponds with the atmospheric pressure value and it is independent on the pressure value inside the luminous tube 1, resp. between their walls and the inserted tube 3 walls. The luminous tube 1 is in that closed space (outside the space of the inserted tube 3) via vacuum flanges first evacuated and subsequently filled with SO molecules. The SO molecules are continuously excited by free electrons which are also present in the space filled by SO molecules, thus the excited SO molecules emit UV radiation.

An object 4 intended for UV radiation treatment is placed inside of the inserted tube 3 and exposed to UV radiation for the time period necessary to the reaching of the requested effect. A reflective casing 5 surrounding the luminous tube 1 enables UV radiation, emitted by SO molecules from the luminous tube 1 through their walls and passing around the object 4, its subsequent reflection and additional interaction with the object 4. The reflective casing 5 also protects the ambient of the device from UV radiation. The reflective casing 5 has a greater diameter than the luminous tube 1 but the gap between the parts is so small (typically just a millimetre) in order to prevent substantial absorption of UV radiation in air between the luminous tube 1 and the reflective casing 5. Such a small gap also makes the device compact. The length of the luminous tube 1 is chosen according to the longitudinal dimensions of the object 4 to be treated. The inner diameter of the inserted tube 3 is typically a few millimetres larger than the transversal dimension of the object 4 so that the object 4 fits in the inserted tube 3. The distance between the outer wall of the inserted tube 3 and the inner wall of the luminous tube 1 is between 10 and 100 mm. The larger distance results in more extensive radiation from SO molecules and thus more intensive treatment of the object 4. Large diameters of the luminous tube 1 are not feasible, thus the typical diameters of luminous tube 1 are in the range from 30 till 300 mm. If the transversal dimension of the object 4 is larger than about 250 mm this embodiment is less suitable and the treatment is better accomplished using embodiment described in Example 3.

The method of producing UV A and UV B radiation using the described device includes following steps:
The luminous tube 1 is evacuated through the vacuum flange 2 and then continuously filled with SO molecules with free electron content. At the same time, the molecules excited by electrons are continuously drained from the opposite end of the luminous tube 1 through the second vacuum flange 2' in such a way that the inner pressure in the luminous tube 1 is kept at the constant value of 1000 Pa. The temperature inside the luminous tube 1 is kept on the value 200°K. Free electrons with the electron density of 10¹⁷ m⁻³ have the average value of their kinetic energy 8 eV and the excited SO molecules can emit UV radiation.

In particular cases (not visible from drawings) the inserted tube 3 is missing and the object 4 is placed directly inside the luminous tube 1. The vacuum flanges 2, 2' are made in such a way to allow for tightening of the luminous tube 1 including the inserted object 4, so that no ambient air enters the luminous tube 1 filled with SO molecules that are excited by electrons in order to emit UV radiation. Such particular cases are typically chosen when the object 4 does not interact chemically with SO molecules or when the interaction is requested.

### Example 2

This embodiment of the device according to the invention is appreciated for the treatment of infinite materials or materials with large longitudinal diameters. The device is schematically shown in the Fig. 2. The luminous tube 1 has a linear shape again and it is made from the material transparent for UV radiation, namely from quartz glass, wherein at both of its ends it is tightly connected to a vacuum flanges 2, 2', which are equipped by a closable inlet/output opening intended for the evacuation and filling/draining of SO molecules with free electron content. The embodiment of this example is similar to the previous one, the only difference lies in the fact that the object 4 of a longitudinal shape is moving through the inserted tube 3 by transporting rollers 6. The length of the object 4 is not limited in this embodiment, however the transversal dimension of the object 4 is limited accordingly to the previous embodiment described in Example 1.

The method of producing UV radiation using the said device is similar to the method already mentioned in Example 1. The place for producing of UV radiation is the luminous tube 1 where the pressure is kept on the constant value 1 Pa and the temperature value is kept constant at 600°K. Free electrons with the electron density of 10¹⁵ m⁻³ have the average value of their kinetic energy 2 eV and thus the excited SO molecules can emit UV radiation in the luminous tube 1.

### Example 3

This embodiment of the device according to the invention, suitable for the treatment of objects 4 with almost arbitrary dimensions, is schematically presented in Figure 3. A set of linear luminous tubes 1 is used there which are made from the material transparent for UV radiation namely quartz glass. The luminous tubes 1 creating UV radiation are mounted parallel each to others inside a reflective casing 5. Typically, there will be 12 luminous tubes 1 there placed longitudinally along the reflective casing 5 as shown schematically in Figure 3. The large object 4 to be treated by UV radiation is positioned and closed between the set of luminous tubes 1 which are surrounded by the reflective casing 5, typically in the central axle of the said space as shown schematically in Figure 3.

The method of producing UV radiation using the described device incudes following steps:
The luminous tubes 1 are first evacuated and then filled with SO molecules with the pressure kept on the value 30 Pa at the temperature value 300°K. The SO molecules are continuously excited by electrons which are also presented in the volume filled by SO gas so the SO molecules emit UV radiation. Free electrons with the electron density of 10¹⁸ m⁻³ have the average value of their kinetic energy 10 eV. The radiation is emitted from each luminous tube 1 into the entire solid angle. The reflective casing 5 visible on the Fig. 3 serves for reflecting the UV radiation, so the treatment efficiency for the large objects 4 is improved several times as compared to a case when the reflective casing 5 is missing. Furthermore, the reflector prevents UV radiation of the ambient so it serves also as a UV protection. Still, in particular cases the reflective casing 5 could be absent.

In this embodiment neither the diameter of the reflective casing 5 nor the luminous tubes 1 are limited, so objects 4 of any size and shape could be treated. The circular cross-section of the reflective casing 5 as shown schematically in Fig. 3 is preferred but the reflective casing 5 could be of any other shape, for example oval or of multi-angled cross-section. The reflective casing 5 could be also made from plates joint together. The length of the luminous tubes 1 is not limited, but feasible embodiments employ the luminous tubes 1 of a length up to few meters. The large object 4 could be also moved or rotated in this embodiment, similar to embodiment described in Example 2.

### Example 4

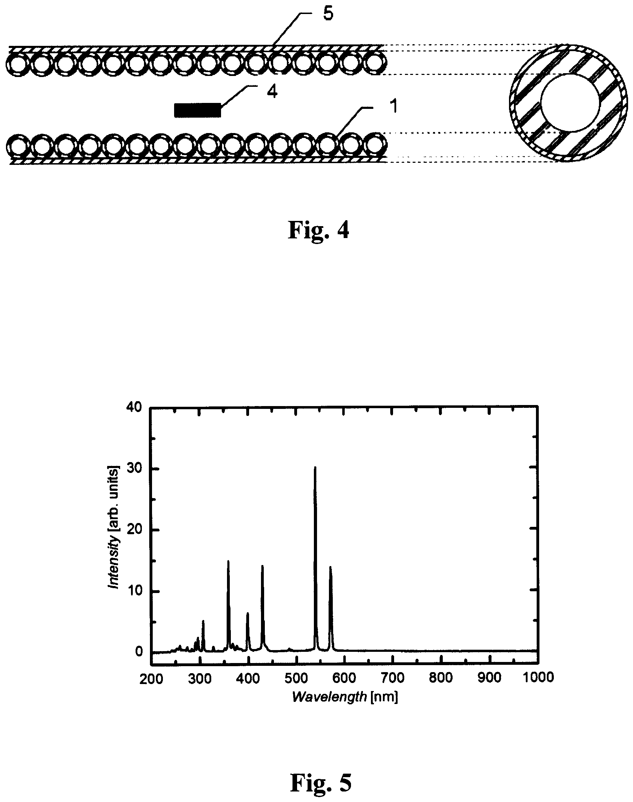

The realization of some embodiments according to the previous Example 3 is limited regarding the length of the device illustrated schematically in the Fig. 3. If the extremely large length of the device is requested, the embodiment of the device according to Example 4 is appreciated, where the length of object 4 being treated is practically unlimited. The said embodiment according to Example 4 is presented schematically in the Fig. 4. In this embodiment, the SO molecules being excited are emitting UV radiation, the said SO molecules are kept together with the free electrons inside the set of toroidal luminous tubes 1 which are mounted at the inner circumference of the reflective casing 5. At least two toroidal luminous tubes 1 are intended there but in the embodiment according to Fig. 4 many such toroidal luminous tubes 1 are used.

The method of producing UV radiation using the described device is realized as follows:
The toroidal luminous tubes 1 are first evacuated and then filled with SO molecules with the pressure kept on the value 100 Pa at the temperature value 350°K. The SO molecules are continuously excited by electrons with the electron density of 10¹⁴ m⁻³ have the average value of their kinetic energy 0,1 eV. The radiation produced by such a way is emitted from each toroidal luminous tube 1 into the entire solid angle. The reflective casing 5 visible on the Fig. 4 serves for reflecting the UV radiation, therefore the treatment efficiency for each object 4 is improved significantly, similarly as in aforesaid embodiments. There is no limitation here in the length of the device since unlimited number of toroidal luminous tubes 1 could be applied. The feasible major diameter of the toroidal luminous tubes 1 is about 1 m.

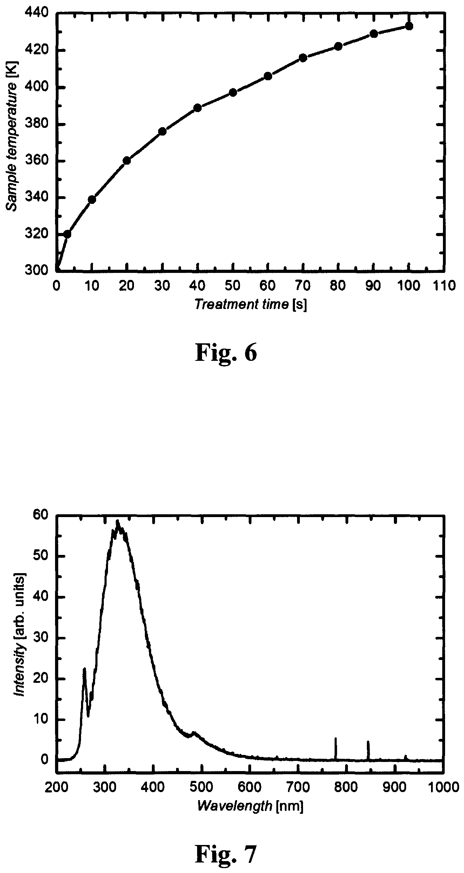

The comparison of the effects of the device according to the invention with the properties of known types of UV sources which are available in the market:
Major drawbacks of commercial UV lamps employing radiation from excited atoms is poor energy efficiency, rather discrete spectrum of radiation and inability to avoid heating of objects exposed to radiation for prolonged time. The drawbacks become apparent from Fig. 5 and 6.

A thin layer of a UV cured coating based on acrylate was deposited on the surface of styrene acrylonitrile sheet (SAN) by a print coat instrument and exposed to radiation from a commercial UV source for curing polymers. The lamp employs mercury vapour as the source of radiation. The spectrum of the lamp was measured with an optical spectrometer. The spectrum is shown in Fig. 5. The most intensive radiation of this lamp does not appear in the UV range, but rather in the visible at about 550, 570 and 440 nm. There is a line in UV A range at 360 nm and a line at the boundary between UV A and visible range at about 400 nm. There is also a line at the boundary between UV A and UV B range - about 315 nm. The temperature of the thin layer of a UV cured coating based on acrylate deposited on the surface of styrene acrylonitrile sheet (SAN) by a print coat instrument was measured with an infra-red pyrometer upon continuous exposure to radiation from the commercial lamp. The polymer sample temperature is plotted in Figure 6 versus the irradiation time.

The temperature increased from the original value before the treatment (i.e. room temperature) for 50°K in less than 20 s, so well before the curing could be accomplished. The heating is predominantly due to absorption of visible radiation from the commercial source. Such a thermal effect is unwanted in practical applications since many materials change the structural and functional properties at such elevated temperatures. The commercial lamp whose spectrum is shown in Figure 1 is therefore unsuitable for prolonged continuous treatment.

Figure 7 shows a spectrum of the device according to the presented invention. The intensity of any atomic line is now marginal as compared to the extensive broad continuum spreading from blue range of spectrum (wavelength of around 500 nm) through the UV A towards UV C range. The continuum peaks roughly at the boundary between UV A and UV B radiation. The absence of extensive radiation at wavelengths other than UV range prevents heating of materials exposed to radiation from the device according to the invention. The temperature of the thin layer of a UV cured coating based on acrylate deposited on the surface of styrene acrylonitrile sheet (SAN) by a print coat instrument was measured with an infra-red pyrometer upon continuous exposure to radiation from the device according to the invention. No deviation from the room temperature did reach the detection limit of the pyrometer employed for determination of the surface temperature of the sample (roughly 5°K) even for treatment times of 10 minutes.

The spectrum shown in Fig. 7 is optimal for curing polymers as well as activation of polymer materials prior to printing, panting, gluing or deposition of a coating. It is also suitable for sterilization of heat-sensitive devices since it contains substantial radiation in the UV C range. Such a spectrum of SO molecules results from appropriate choice of energy of free electrons used for excitation of the SO radiative states. The electron energy should be high enough to enable excitation and low enough to prevent extensive dissociation of the SO molecules to the parent atoms. The dissociation energy of the SO molecule is 5.3 eV, what is also the threshold energy for electrons to be capable of dissociating the molecule. The probability for dissociation then increases steeply with increasing electron energy, so that electrons of the energy above 10 eV will dissociate the molecule rather than excite. In the embodiment used to measure the spectrum in Fig. 7 the electrons were of Maxwellian distribution over kinetic energy with the temperature of about 4 eV. A substantial fraction of electrons is therefore capable of exciting SO molecules to states radiating in the UV range (400 nm corresponds to 3.1 eV) but less than 1% has the energy above 10 eV where the dissociation becomes significant.

The optimal distribution of electrons over kinetic energy would be discrete with the energy just below the dissociation threshold, but such a solution is not feasible. The device according to the invention thus employs electrons of distribution close to Maxwellian. To obtain the spectrum shown in Figure 7 the electrons were almost Maxwellian with the temperature of 4 eV. The electron density was about 1x10¹⁶ m⁻³ which is a value high enough to assure for extensive excitation to radiative states but not sufficient to dissociate the SO molecules extensively. Such electron temperature and density can be obtained by continuous heating of electrons in the gas phase by an appropriate electrical field. Both DC and AC field can be applied. In the preferred embodiment the AC field is applied.

### INDUSTRIAL APPLICABILITY

The device for producing of UV radiation and the method for producing such radiation characterized by very stable heat regime is recommended for preferable using for the activation or curing of heat sensitive polymers. The invention can be also successfully applied for the deactivation of bacteria, viruses and fungal spores as well as for the surface sterilisation of some objects and materials. The gentle heat regime without overheating, required for the treatment of all heat sensitive materials, will find employment here, too.

### List of numbered items on drawings:

**1 - luminous tube**
**2, 2'- vacuum flanges**
**3 - inserted tube**
**4 - object** (to be treated)
**5 - reflective casing**
**6 - transporting rollers**

## Claims

1. A device for producing UV radiation, providing preferably UV A and UV B radiation with negligible amount of radiation in the visible or near IR range, said device comprising at least one luminous tube which is closed against ambient air and whose walls are transparent for UV A and UV B radiation, said luminous tube containing sulphur oxide molecules, the device being **characterized by** means for a continuous supply of sulphur oxide molecules with a content of free electrons into said luminous tube (1), such that said luminous tube (1) contains a stable amount of sulphur oxide (SO) molecules, and a stable amount of free electrons.

2. Device according to claim 1 **w her e i n** the luminous tube (1) has a linear shape and it is closed at its ends by vacuum flanges (2, 2') which are equipped by either input or outlet opening for sulphur oxide with the content of free electrons.

3. Device according to claims 1 and 2 **w her e i n** the luminous tube (1) contains a coaxially placed inserted tube (3) which passes through at least one vacuum flange (2) wherein the walls of the said inserted tube (3) are transparent for UV A and UV B radiation, and the device is so adapted that an object (4) can be placed inside the inserted tube (3), the said object (4) is intended for the treatment by UV A and UV B radiation.

4. Device according to claim 1 **wherein** it has a reflective casing (5) which is created from UV radiation reflecting material.

5. Device according to claims 1 and 3 **wherein** the inserted tube (3) passes through both of vacuum flanges (2, 2') and there are transporting rollers (6) placed near to vacuum flanges (2, 2'), the said transporting rollers (6) are adapted for movement of the object (4) which longitudinal dimension exceeds the relevant dimension of the device, through the inserted tube (3).

6. Device according to claim 1 wherein it comprises at least two luminous tubes (1), advantageously a set of luminous tubes (1) which are adapted to be spaced around the object (4) intended for the treatment by UV A and UV B radiation.

7. Device according to claims 1 and 6 **wherein** the luminous tubes (1) are of a toroidal shape.

8. Method for producing of UV radiation using the device according to claim 1 **wherei n** the luminous tube (1) is filled by sulphur oxide (SO) molecules with stable amount of free electrons, wherein the SO molecules are supplied and drained inside/outside the luminous tube (1) in such a way that the pressure inside the luminous tube (1) as well as the amount of free electrons remains constant.

9. Method for producing of UV radiation using the device according to claim 8, **where in** the luminous tube (1) contains SO molecules with temperature between 200 and 600 K, preferably between 300 and 350 K.

10. Method for producing of UV radiation using the device according to claim 8, **where in** the luminous tube (1) contains SO molecules with pressure between 1 and 10000 Pa, preferably between 30 and 100 Pa.

11. Method for producing of UV radiation using the device according to claim 8, **where in** the luminous tube (1) contains free electrons having the average kinetic energy between 0.1 and 10 eV, preferably between 2 and 8 eV.

12. Method for producing of UV radiation using the device according to claim 8, **where** in the luminous tube (1) contains free electrons having the density between 10¹⁴ and 10¹⁸ m⁻³, preferably between 10¹⁵ and 10¹⁷ m⁻³.

## Patentansprüche

1. Vorrichtung zur Erzeugung von UV-Strahlung, die vorzugsweise UV-A- und UV-B-Strahlung mit vernachlässigbarer Strahlungsmenge im sichtbaren oder nahen IR-Bereich bereitstellt, umfassend mindestens eine gegen Umgebungsluft geschlossene Leuchtröhre, deren Wände für UV-A- und UV-B-Strahlung durchlässig sind, wobei die Leuchtröhre Schwefeloxidmoleküle enthält, die Vorrichtung ist **gekennnzeichnet** durch Mittel zur kontinuierlichen Zuführung von Schwefeloxidmolekülen mit einem Gehalt an freien Elektronen in die Leuchtröhre (1), so dass die Leuchtröhre (1) eine stabile Menge an Schwefeloxid (SO)-Molekülen und eine stabile Menge an freien Elektronen enthält.

2. Vorrichtung nach dem Anspruch 1, **wobei** die Leuchtröhre (1) eine lineare Form aufweist und an ihren Enden durch Vakuumflansche (2, 2'), die entweder mit einer Ein- oder Austrittsöffnung für Schwefeloxid mit dem Gehalt an freien Elektronen ausgestattet sind, verschlossen ist.

3. Vorrichtung nach den Ansprüchen 1 und 2, **wobei** die Leuchtröhre (1) ein koaxial angeordnetes Einsatzrohr (3) enthält, das mindestens einen Vakuumflansch (2) durchsetzt, wobei die Wände des eingesetzten Rohres (3) für UV-A- und UV-B-Strahlung durchlässig sind, und die Vorrichtung so eingerichtet ist, dass ein Gegenstand (4) innerhalb des eingesetzten Rohres (3) platzierbar ist, wobei der Gegenstand (4) für die Behandlung durch UV-A- und UV-B-Strahlung vorgesehen ist.

4. Vorrichtung nach dem Anspruch 1, **wobei** sie eine reflektierende Umhüllung (5) aufweist, die aus UV-Strahlung reflektierendem Material gebildet ist.

5. Vorrichtung nach den Ansprüchen 1 und 3, **wobei** das eingesetzte Rohr (3) beide Vakuumflansche (2, 2') durchsetzt und Transportrollen (6) vorhanden sind, die in der Nähe der Vakuumflansche (2, 2') angeordnet sind, wobei die Transportrollen (6) zur Bewegung des Gegenstandes (4), dessen Längsgröße die entsprechende Größe der Vorrichtung übersteigt, durch das eingesetzte Rohr (3).

6. Vorrichtung nach dem Anspruch 1, **wobei** sie mindestens zwei Leuchtröhren (1), vorteilhaft einen Satz von Leuchtröhren (1), umfasst, die dazu eingerichtet sind, um rund um den für die Behandlung durch UV-A- und UV-B-Strahlung vorgesehenen Gegenstand (4) in Abständen angeordnet zu sein.

7. Vorrichtung nach den Ansprüchen 1 und 6, **wobei** die Leuchtröhre (1) kreisringförmig sind.

8. Verfahren zur Bildung von UV-Strahlung unter Verwendung der Vorrichtung nach dem Anspruch 1, **wobei** die Leuchtröhre (1) mit Schwefeloxid (SO)-Molekülen mit einer stabilen Menge an freien Elektronen gefüllt ist, wobei die SO-Moleküle innerhalb/außerhalb der Leuchtröhre (1) derart zugeführt und abgeleitet werden, so dass der Druck innerhalb der Leuchtröhre (1) sowie die Menge an freien Elektronen konstant bleiben.

9. Verfahren zur Bildung von UV-Strahlung unter Verwendung der Vorrichtung nach dem Anspruch 8, **wobei** die Leuchtröhre (1) SO-Moleküle mit einer Temperatur zwischen 200 und 600 K, vorzugsweise zwischen 300 und 350 K, enthält.

10. Verfahren zur Bildung von UV-Strahlung unter Verwendung der Vorrichtung nach dem Anspruch 8, **wobei** die Leuchtröhre (1) SO-Moleküle mit einem Druck zwischen 1 und 10000 Pa, vorzugsweise zwischen 30 und 100 Pa, enthält.

11. Verfahren zur Bildung von UV-Strahlung unter Verwendung der Vorrichtung nach dem Anspruch 8, **wobei** die Leuchtröhre (1) freie Elektronen mit der mittleren kinetischen Energie zwischen 0,1 und 10 eV, vorzugsweise zwischen 2 und 8 eV, enthält.

12. Verfahren zur Bildung von UV-Strahlung unter Verwendung der Vorrichtung nach dem Anspruch 8, **wobei** die Leuchtröhre (1) freie Elektronen mit der Dichte zwischen 10¹⁴ und 10¹⁸ m⁻³, vorzugsweise zwischen 10¹⁵ und 10¹⁷ m⁻³, enthält.

## Revendications

1. Dispositif de production de rayonnement UV, fournissant de préférence un rayonnement UV A et UV B avec une quantité négligeable de rayonnement dans la plage visible ou proche IR, ledit dispositif comprenant au moins un tube lumineux qui est fermé contre l'air ambiant et dont les parois sont transparentes pour le rayonnement UV A et UV B, ledit tube lumineux contenant des molécules d'oxyde de soufre, le dispositif étant **caractérisée par** des moyens pour une alimentation continue de molécules d'oxyde de soufre avec une teneur en électrons libres dans ledit tube lumineux, de telle sorte que ledit tube lumineux (1) contient une quantité stable de molécules d'oxyde de soufre (SO), et une quantité stable d'électrons libres.

2. Dispositif selon la revendication 1, **dans lequel** le tube lumineux (1) a une forme linéaire et il est fermé à ses extrémités par des brides sous vide (2, 2') qui sont dotées soit d'une ouverture d'entrée soit d'une ouverture de sortie pour l'oxyde de soufre avec la teneur en électrons libres.

3. Dispositif selon les revendications 1 et 2, **dans lequel** le tube lumineux (1) contient un tube introduit coaxialement (3) qui traverse au moins une bride à vide (2), les parois dudit tube inséré (3) étant transparentes pour un rayonnement UV A et UV B, et le dispositif est conçu de telle sorte qu'un objet (4) peut être placé à l'intérieur du tube inséré (3), ledit objet (4) étant destiné au traitement par rayonnement UV A et UV B.

4. Dispositif selon la revendication 1, **dans lequel** il présente un boîtier réfléchissant (5) qui est formé à partir d'un matériau réfléchissant les rayons UV.

5. Dispositif selon les revendications 1 et 3, **dans lequel** le tube inséré (3) passe à travers les deux brides à vide (2, 2') et il y a des rouleaux de transport (6) placés à proximité des brides à vide (2, 2'), lesdits rouleaux de transport (6) étant conçus pour le déplacement de l'objet (4), la dimension longitudinale duquel dépasse la dimension correspondante du dispositif, à travers le tube inséré (3).

6. Dispositif selon la revendication 1, **lequel** comprennant au moins deux tubes lumineux (1), avantageusement un ensemble de tubes lumineux (1) qui sont conçus pour être espacés autour de l'objet (4) destiné au traitement par rayonnement UV A et UV B.

7. Dispositif selon les revendications 1 et 6, **dans lequel** les tubes lumineux (1) ont une forme torique.

8. Procédé de production d'un rayonnement UV à l'aide du dispositif selon la revendication 1, **caractérisée par** le tube lumineux (1) étant rempli par des molécules de l'oxyde de soufre (SO) ayant une quantité stable d'électrons libres, les molécules SO étant alimentées et drainées à l'intérieur/à l'extérieur du tube lumineux (1) de telle sorte que la pression à l'intérieur du tube lumineux (1) ainsi que la quantité d'électrons libres restent constantes.

9. Procédé de production d'un rayonnement UV à l'aide du dispositif selon la revendication 8, **dan s lequel** le tube lumineux (1) contient des molécules SO avec une température comprise entre 200 et 600 K, de préférence entre 300 et 350 K.

10. Procédé de production d'un rayonnement UV à l'aide du dispositif selon la revendication 8, **dans lequel** le tube lumineux (1) contient des molécules SO avec une pression comprise entre 1 et 10000 Pa, de préférence entre 30 et 100 Pa.

11. Procédé de production d'un rayonnement UV à l'aide du dispositif selon la revendication 8, **dans lequel** le tube lumineux (1) contient des électrons libres ayant l'énergie cinétique moyenne comprise entre 0,1 et 10 eV, de préférence entre 2 et 8 eV.

12. Procédé de production d'un rayonnement UV à l'aide du dispositif selon la revendication 8, **dans lequel** le tube lumineux (1) contient des électrons libres ayant une densité comprise entre 10¹⁴ et 10¹⁸ m⁻³, de préférence entre 10¹⁵ et 10¹⁷ m⁻³.
